# EUROPÄISCHE PATENTSCHRIFT

(11) **EP 0 630 886 B1**
(45) Veröffentlichungstag und Bekanntmachung des Hinweises auf die Patenterteilung: **22.04.1998**
(21) Anmeldenummer: 94109478.1
(22) Anmeldetag: 20.06.1994
(51) Int. Cl.: C07C 255/24, C07C 253/30

(54) **Verfahren zur Herstellung von Aminopropionitrilen**
Process for the preparation of aminopropionitriles
Procédé pour la préparation d'aminopropionitriles

(30) Priorität: 28.06.1993 DE 4321273
(43) Veröffentlichungstag der Anmeldung: 28.12.1994
(73) Patentinhaber: BASF Aktiengesellschaft, 67063 Ludwigshafen (DE)
(72) Erfinder: Witzel, Tom, Dr., D-67069 Ludwigshafen (DE); Kummer, Rudolf, Dr., D-67227 Frankenthal (DE); Merger, Franz, Dr., D-67227 Frankenthal (DE); Voit, Guido, Dr., D-69198 Schriesheim (DE); Brudermueller, Martin, Dr., D-68165 Mannheim (DE); Priester, Claus-Ulrich, Dr., D-67065 Ludwigshafen (DE); Harder, Wolfgang, Dr., D-69469 Weinheim (DE)

(56) Entgegenhaltungen:
- EP-A- 0 352 504
- DE-A- 2 436 651
- DE-C- 598 185
- US-A- 4 211 725

## Beschreibung

Die vorliegende Erfindung betrifft ein neues und verbessertes Verfahren zur Herstellung von Aminopropionitrilen durch Umsetzung von überschüssigem Ammoniak mit Acrylnitrilen an Heterogenkatalysatoren bei erhöhten Temperaturen und erhöhten Drücken.

Aus J. Chem. Soc., 1369 bis 1371 (1947) ist die Umsetzung von ω-Bromalkylcyaniden mit dem Kaliumsalz des Phthalsäureimids und nachfolgende Reaktion mit Hydrazinhydrat beschrieben. Dieses Verfahren ist z.B. zur technischen Herstellung von 3-Aminopropionsäurenitril aber ungeeignet.

Aus der US-A 3 174 992 ist die Umsetzung von Ethylencyanhydrin mit Ammoniak an feuchtem Raney-Nickel bei Temperaturen um 100°C unter Eigendruck bekannt. Die Reaktion verläuft mit 54 % Ausbeute unbefriedigend.

3-Aminopropionsäurenitril und Bis-(2-cyanethyl)amin erhält man im ungünstigen Verhältnis 2/1 auch auf dem technisch aufwendigen Umweg über 2-Methoxypropionsäurenitril durch Umsetzung mit Ammoniak in Gegenwart von Raney-Cobalt bei Temperaturen von 65 bis 80°C und 165 bar Druck nach DE-A-10 03 740.

Aus der US-A-4 211 725 ist bekannt, daß man substituierte Aminopropionitrile mit flüssigem Ammoniak mit geeigneten Metallkatalysatoren - wie beispielsweise Raney-Nickel, Ru, Co, Fe, Pd, Pt, Os, Ir, Rh entweder als Metall oder als Metall aufgebracht auf einem inerten Träger - erhalten kann.

Zur Herstellung von 3-Aminopropionsäurenitril durch Direktumsetzung von Acrylnitril mit Ammoniak ist bekannt, daß wasserfreies Ammoniak bei Raumtemperatur nicht mit Acrylnitril reagiert, sondern vielmehr als Stabilisator für Acrylnitril eingesetzt werden kann (US-A 2 432 511). Aus der US-A 2 401 429 ist bekannt, daß man nach 2 Tagen aus Acrylnitril und flüssigem Ammoniak bei Raumtemperatur neben 76 % Bis-(2-cyanethyl)ether auch 11 % 3-Aminopropionsäurenitril isolieren kann. Bei 90°C setzt sich Acrylnitril unter Druck mit flüssigem Ammoniak zu 12,5 % 3-Aminopropionitril um (DE-A-598 185).

Ferner ist bekannt, daß sich der Zusatz von protischen Lösungsmitteln vorteilhaft auf die Addition von NH₃ an Acrylnitril auswirkt. Der Zusatz von Wasserdampf zum Gemisch aus Acrylnitril-Ammoniak ist beispielsweise aus der Chem. Abstr. Vol. 83, 26879 bekannt. Üblicherweise wird jedoch wäßriger Ammoniak im Temperaturbereich von 80 bis 130°C eingesetzt. Bei einem Verhältnis von Ammoniak/Acrylnitril/Wasser 5 bis 15 : 1 : 5 bis 20 erhält man 3-Aminopropionitril neben Bis-(2-cyanethyl)amin in Ausbeuten von 57 bis 80 % (z.B. US-A 3 935 256 -62 %, DE-A-24 36 651 -70 %,

US-A 2 44.8 013 -78 %, US-A-2 019 903 -59 %, Org. Syn. 27, 3 bis 5 (1947) -57 %). Nachteile dieser Verfahren unter Einsatz von wässrigem Ammoniak ergeben sich bei der Auf- bzw. Weiterverarbeitung des Produktgemisches:
- Destillative Entfernung von zugesetztem Wasser bei der in Folge großer Ammoniakmengen nötigen Kreislauffahrweise
- Selektivitätsverluste für 3-Aminopropionsäurenitril bei der Abtrennung von Ammoniak/Wasser
- Hydrolyse der Nitrilgruppen
- Katalysatorschädigung bei der nachfolgenden Hydrierung.

Aus Przemyst. Chem. 44(2), 85 (1965) bzw. GB-A-642 409 sind Verfahren bekannt, bei denen durch Zusatz von 15 bis 20 Äquivalenten Methanol eine Ausbeute von 81 % 3-Aminopropionitril erzielt werden. Die Bildung von Nebenprodukten durch Methylierung wirkt prohibitiv für die technische Nutzung des Verfahrens. Eine Ausbeute von 68 % 3-Aminopropionitril erhält man hingegen bei Zusatz von 3 Äquivalenten tert.-Butanol (US-A 2 742 491).

Die in DE-A-24 36 651 beschriebene Herstellung von 3-Aminopropionitril durch Aminolyse von Bis-(2-cyanethyl)amin mit Ammoniak verlangt Temperaturen von 130 bis 170°C und verläuft sehr langsam mit Reaktionszeiten von bis zu 165 min.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminopropionitrilen der allgemeinen Formel I 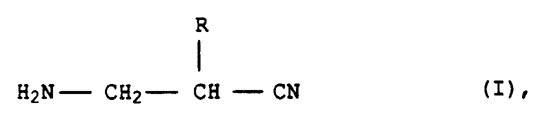
- R: Wasserstoff oder Methyl
bedeutet, welches dadurch gekennzeichnet ist, daß man Ammoniak mit Acrylnitrilen der allgemeinen Formel II 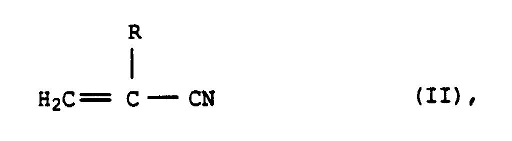 in der die Substituenten die oben genannten Bedeutungen haben, im Molverhältnis von 1:1 bis 500:1 an Oxiden der 7. oder 8. Nebengruppe des Periodensystems der Elemente, sauren oder basischen organischen Ionenaustauschern oder Gemischen aus diesen als Heterogenkatalysatoren bei Temperaturen von 40 bis 180°C und Drükken von 10 bis 350 bar umsetzt.

Das erfindungsgemäße Verfahren läßt sich wie folgt durchführen:

Die Reaktion kann bei Temperaturen von 40 bis 180°C und Drücken von 10 bis 350 bar diskontinuierlich oder vorzugsweise kontinuierlich durchgeführt werden. Das bevorzugte kontinuierliche Verfahren kann bei Temperaturen von 40 bis 150°C und Drücken von 10 bis 200 bar, vorzugsweise bei 50 bis 120°C und 150 bis 230 bar durchgeführt werden.

Üblicherweise wird Ammoniak mit 0 bis 5 Gew.-% Wasser, bevorzugt im wesentlichen wasserfrei (0 bis 1 Gew.-% Wasser) besonders bevorzugt mit 0,1 - 1 Gew.-% Wasser mit den Acrylnitrilen im Molverhältnis von 1:1 bis 500:1, bevorzugt 2:1 bis 100:1, besonders bevorzugt 10:1 bis 80:1 eingesetzt. In der Reaktion nichtumgesetzter Ammoniak kann ohne Verschlechterung der Aminopropionitril-Ausbeute technisch einfach in die Umsetzung zurückgeführt werden (Kreislauffahrweise).

Im allgemeinen verwendet man keine Lösungsmittel; man kann jedoch gegebenenfalls inerte Lösungsmittel z.B. Ether wie Dibutylether, Tetrahydrofuran, Dimethoxiether, oder z.B. Kohlenwasserstoffe wie Cyclohexan, Benzol und Toluol in Mengen von 0 bis 500 % Gew.%, bevorzugt 50 bis 200 Gew.-% zum Acrylnitril II einsetzen.

Bei der Umsetzung hält man vorteilhaft eine Katalysatorbelastung von 0,1 bis 10 g, vorzugsweise 0,1 bis 2 g Acrylnitril je g Katalysator und Stunde ein.

Als Heterogenkatalysatoren eignen sich sauer und/oder basisch bzw. amphoter wirkende Oxide von Elementen der siebten und achten Nebengruppe, wie Fe₂O₃, MnO₂, Co₃O₄, Mn₂O₃, MnO, Re₂O₇, Fe₃O₄, CoO, NiO und , bevorzugt MnO₂, Fe₂O₃ und Co₃O₄ besonders bevorzugt Fe₂O₃.

Weitere Katalysatoren für das erfindungsgemäße Verfahren sind saure oder basische Ionenaustauscher wie Gel-Ionenaustauscher auf Basis Styrol wie z.B. solche der Marken Lewatit® oder Amberlite®, makroporöse Ionenaustauscher auf Basis Styrol wie z.B. solche der Marke Lewatit®, makroretikulare Ionenaustauscher auf Basis Styrol oder Acryl wie z.B. solche der Marke Amberlite® oder makroporöse Austauscher auf Basis Siloxan wie z.B. solche der Marke Deloxan®, besonders bevorzugt makroporöse Ionenaustauscher.

Die in diesem Verfahren verwendeten Aminopropionitrile der allgemeinen Formel I sind:
3-Aminopropionsäurenitril,
3-Amino-isobutyronitril.

Die nach dem erfindungsgemäßen Verfahren herstellbaren Aminopropionitrile der allgemeinen Formel I sind Zwischenprodukte für die Herstellung von Diaminen, Aminocarbonsäuren bzw. Aminocarbonsäureamiden.

Das nach dem erfindungsgemäßen Verfahren herstellbare 3-Aminopropionitril der allgemeinen Formel I eignet sich als Zwischenprodukt für die Herstellung von
- β-Aminosäuren: β-Alanin --->
   Vorprodukt für Calciumpanthothenat
   durch Hydrolyse (DE-A-22 23 236)
- 1,3-Propylendiamin, die Verwendung in Pharmazeutika, Polyamiden und Holzschutzmitteln finden (DE-A 32 48 326, DT 20 04 405)

### Beispiele

### Beispiel 1

Durch einen mit 51 g Fe₂O₃ (1 - 2 mm Split) gefüllten Rohrreaktor wurde bei 50°C und einem Druck von 150 bar ein Gemisch aus 210 ml flüssigem Ammoniak und 19 ml Acrylnitril (Belastung 0,3 g Acrylnitril g⁻¹ Kat. h⁻¹) gepumpt. Der Umsatz betrug 90 %. Nach 22 h Betriebsdauer wies der Reaktionsaustrag folgende Zusammensetzung auf (quant.GC):
57,6 Gew.-% 3-Aminopropionitril
33,1 Gew.-% Bis-(2-cyanethyl)amin
8 Gew.-% Acrylnitril

### Beispiel 2

Durch einen mit 29 g sauren Ionenaustauscher (Amberlyst 15) gefüllten Rohrreaktor wurden bei 50°C und einem Druck von 180 bar ein Gemisch aus 215 ml flüssigen Ammoniak und 19 ml Acrylnitril (Belastung 0,53 g Acrylnitril g⁻¹ Kat. h⁻¹) gepumpt. Der Umsatz betrug 99,4 %. Nach 20 h Betriebsdauer wies der Reaktionsaustrag folgende Zusammensetzung auf (quant. GC):
81,0 Gew.-% 3-Aminopropionitril
18,2 Gew.-% Bis-(2-cyanethyl)amin
0,48 Gew.-% Acrylnitril

### Beispiel 3

Durch einen mit 36 g basischen Ionenaustauscher (Lewatit MP-600) gefüllten Rohrreaktor wurden bei 50°C und einem Druck von 180 bar ein Gemisch aus 215 ml flüssigem Ammoniak und 19 ml Acrylnitril (Belastung 0,42 g Acrylnitril g⁻¹ Kat. h⁻¹) gepumpt. Der Umsatz betrug 99,4 %. Nach 20 h Betriebsdauer wies der Reaktionsaustrag folgende Zusammensetzung auf (quant. GC):
77,8 Gew.-% 3-Aminopropionitril
20,2 Gew.-% Bis-(2-cyanethyl)amin
0,48 Gew.-% Acrylnitril

## Patentansprüche

1. Verfahren zur Herstellung von Aminopropionitrilen der allgemeinen Formel I 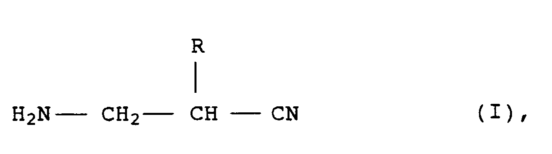 in der
R Wasserstoff oder Methyl
bedeutet, dadurch gekennzeichnet, daß man Ammoniak mit Acrylnitrilen der allgemeinen Formel II 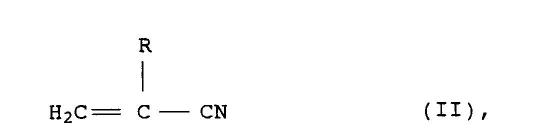 in der die Substituenten die oben genannten Bedeutungen haben, im Molverhältnis von 1:1 bis 500:1 an Oxiden der 7. oder 8. Nebengruppe des Periodensystems der Elemente, sauren oder basischen organischen Ionenaustauschern oder Gemischen aus diesen als Heterogenkatalysatoren bei Temperaturen von 40 bis 180°C und Drücken von 10 bis 350 bar umsetzt.

2. Verfahren zur Herstellung von Aminopropionitrilen nach Anspruch 1, dadurch gekennzeichnet, daß R Wasserstoff bedeutet.

3. Verfahren zur Herstellung von Aminopropionitrilen nach Anspruch 1, dadurch gekennzeichnet, daß man als Heterogenkatalysatoren saure oder basische organische Ionenaustauscher verwendet.

4. Verfahren zur Herstellung von Aminopropionitrilen nach Anspruch 1, dadurch gekennzeichnet, daß man Ammoniak mit den Acrylnitrilen II im Molverhältnis von 10:1 bis 80:1 einsetzt.

5. Verfahren zur Herstellung von Aminopropionitrilen nach Anspruch 1, dadurch gekennzeichnet, daß man die Umsetzung bei Temperaturen von 50 bis 120°C durchführt.

6. Verfahren zur Herstellung von Aminopropionitrilen nach Anspruch 1, dadurch gekennzeichnet, daß man die Umsetzung bei Drücken von 150 bis 230 bar durchführt.

## Claims

1. A process for the preparation of an aminopropionitrile of the formula I 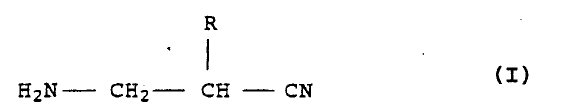 where R is hydrogen or methyl, wherein ammonia is reacted with an acrylonitrile of the formula II 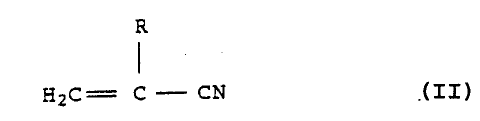 where the substituents have the abovementioned meanings, in a molar ratio of from 1 : 1 to 500 : 1 over an oxide of the seventh or eighth subgroup of the Periodic Table of Elements, an acidic or basic organic ion exchanger or a mixture thereof as a heterogeneous catalyst at from 40 to 180°C and from 10 to 350 bar.

2. A process for the preparation of an aminopropionitrile as claimed in claim 1, wherein R is hydrogen.

3. A process for the preparation of an aminopropionitrile as claimed in claim 1, wherein the heterogeneous catalyst used is an acidic or basic organic ion exchanger.

4. A process for the preparation of an aminopropionitrile as claimed in claim 1, wherein ammonia is used with an acrylonitrile II in a molar ratio of from 10 : 1 to 80 : 1.

5. A process for the preparation of an aminopropionitrile as claimed in claim 1, wherein the reaction is carried out at from 50 to 120°C.

6. A process for the preparation of an aminopropionitrile as claimed in claim 1, wherein the reaction is carried out at from 150 to 230 bar.

## Revendications

1. Procédé de préparation d'aminopropionitriles de formule générale I 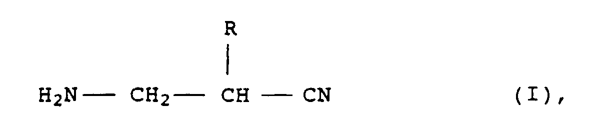 dans laquelle
R représente un atome d'hydrogène ou un groupement méthyle, caractérisé en ce que l'on fait réagir de l'ammoniac avec des acrylonitriles de formule générale II 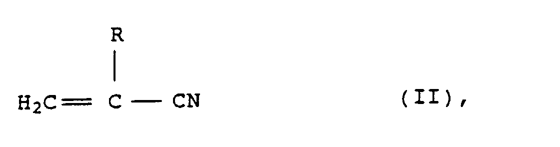 dans laquelle les substituants prennent la signification susmentionnée, dans un rapport en moles de 1:1 à 500:1 en présence d'oxydes d'éléments des groupes VIIb ou VIII de la classification périodique des éléments, d'échangeurs d'ions organiques acides ou basiques ou de mélanges de ceux-ci, en tant que catalyseurs hétérogènes à des températures de 40 à 180°C et sous des pressions de 10 à 350 bar.

2. Procédé de préparation d'aminopropionitriles selon la revendication 1, caractérisé en ce que R représente un atome d'hydrogène.

3. Procédé de préparation d'aminopropionitriles selon la revendication 1, caractérisé en ce que l'on utilise en tant que catalyseurs hétérogènes des échangeurs d'ions organiques acides ou basiques.

4. Procédé de préparation d'aminopropionitriles selon la revendication 1, caractérisé en ce que l'on utilise de l'ammoniac avec les acrylonitriles II dans un rapport en moles de 10:1 à 80:1.

5. Procédé de préparation d'aminopropionitriles selon la revendication 1, caractérisé en ce que l'on effectue la réaction à des températures de 50 à 120°C.

6. Procédé de préparation d'aminopropionitriles selon la revendication 1, caractérisé en ce que l'on effectue la réaction sous des pressions de 150 à 230 bar.
